# EUROPÄISCHE PATENTANMELDUNG

(11) **EP 1 378 235 A1**
(43) Veröffentlichungstag der Anmeldung: **07.01.2004**
(21) Anmeldenummer: 02014838.3
(22) Anmeldetag: 03.07.2002
(51) Int. Cl.: A61K 31/138, A61P 25/18

(54) **Metropolol zur Behandlung von Krankheiten des schizophrenen Formenkreises**

(71) Anmelder: Pro Science Private Research Clinic GmbH, 35440 Linden (DE)
(72) Erfinder: Dimpfel, Wilfried, Prof. Dr., Pro Science Private, 35440 Linden (DE)
(74) Vertreter: HOFFMANN - EITLE

(57) **Zusammenfassung**

Die vorliegende Erfindung betrifft die Verwendung von Metropolol oder seinen pharmazeutisch annehmbaren Salzen zur Herstellung eines Arzneimittels zur Behandlung von Krankheiten des schizophrenen Formenkreises.

## Beschreibung

### Technisches Gebiet

Die vorliegende Erfindung betrifft die Verwendung von Metropolol sowie dessen pharmazeutisch annehmbaren Salzen zur Herstellung eines Arzneimittels zur Behandlung von Krankheiten des schizophrenen Formenkreises.

### Hintergrund der Erfindung

Metropolol, auch als (RS)-Isopropylamino-3-[4-(2-methoxyethyl)phenoxy]-2-propanol bekannt ist ein sogenannter β1(adreno)-selektiver-Rezeptorblocker mit der folgenden allgemeinen Formel:

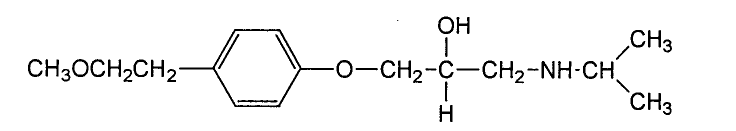

Derzeit ist Metropolol als Antihypertensivum auf dem Markt. Darüber hinaus wird Metropolol zur Behandlung von anginösen Beschwerden sowie im Bereich koronarer Herzkrankheiten eingesetzt. In zunehmendem Maße wird das Präparat auch zur Migräneprophylaxe empfohlen (P. Tfelt-Hansen (1986) "Efficacy of beta-blockers in migraine. A critical review." Cephalalgia 6, 15-24). Die Substanz überwindet offensichtlich die Blut-Hirn-Schranke. Auf der Basis der bisher bekannt gewordenen Untersuchungen war eine Wirkung von Metropolol auf das Krankheitsbild der Schizophrenie nicht zu erwarten gewesen. Erst die Anwendung der völlig "bias"-freien Untersuchung im Tele-Stereo-EEG der Ratte erbrachte den Beweis des Vorhandenseins einer derartigen Wirkung.

Die derzeit üblichen Arzneimittel zur Behandlung von Schizophrenie sind unzufriedenstellend, da sie meist schwerwiegende Nebenwirkungen besitzen. So haben viele antipsychotisch wirkende Arzneimittel kurzfristige (Parkinsonähnliche Symptomatik) und langfristige Nebenwirkungen (z.B. tardive Dyskinesien). Da die Therapie psychotischer Erkrankungen meist lebenslang erfolgen muss, besteht ein großer Bedarf nach einem verbesserten Arzneimittel zur Behandlung des schizophrenen Formenkreises, insbesondere eines Arzneimittels mit einer guten Wirksamkeit bei gleichzeitig geringeren Nebenwirkungen als bei den bekannten Arzneimitteln. Die Gruppe der β-Rezeptorblocker (insbesondere Metropolol) wird seit Jahrzehnten millionenfach verschrieben und zeichnet sich generell durch eine gute Verträglichkeit aus.

### Zusammenfassung der Erfindung

Diese Aufgabe wurde überraschenderweise durch die Verwendung von Metropolol bzw. dessen pharmazeutisch annehmbaren Salzen als Wirkstoff für die Herstellung eines Arzneimittels zur Behandlung von Schizophrenien gelöst.

### Kurze Beschreibung der Abbildungen

Fig. 1 zeigt die Veränderungen der EEG-Frequenzen bei der Ratte nach Gabe klassischer Arzneimittel zur Behandlung von Schizophrenien (Neuroleptika) im Vergleich zu Metropolol.

Fig. 2 zeigt eine Diskriminanzanalyse der EEG-Frequenzänderungen nach Gabe von Medikamenten für unterschiedliche Indikationen.

Fig. 3a und 3b zeigen die zeit- und dosisabhängigen Veränderungen der EEG-Frequenzen bei der Ratte nach Gabe von Metropolol.

### Detaillierte Beschreibung der Erfindung

Bei Untersuchungen der Veränderungen der EEG-Frequenzen der Ratte nach Gabe von Metropolol wurde überraschenderweise gefunden, dass das Präparat eine Wirkung im Gehirn besitzt, die bei der Behandlung von psychotischen Zuständen nützlich ist.

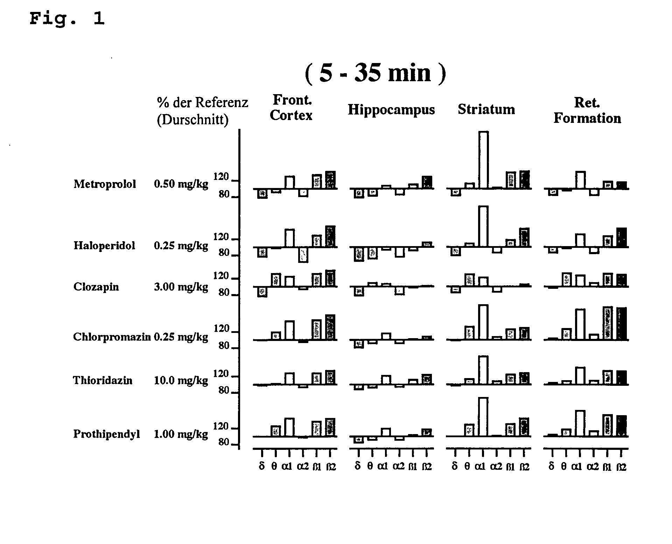

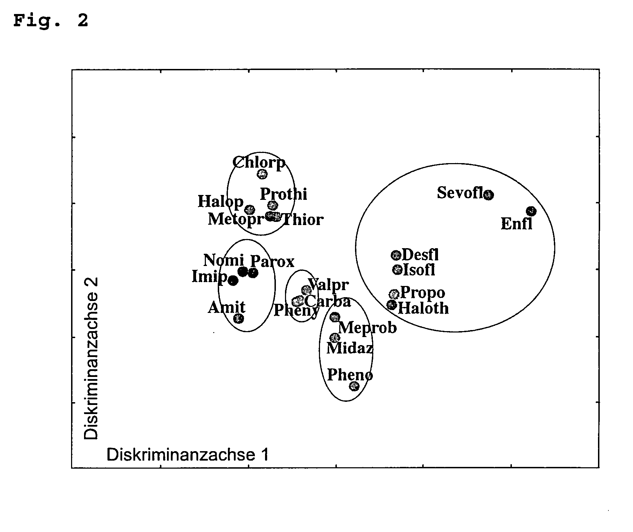

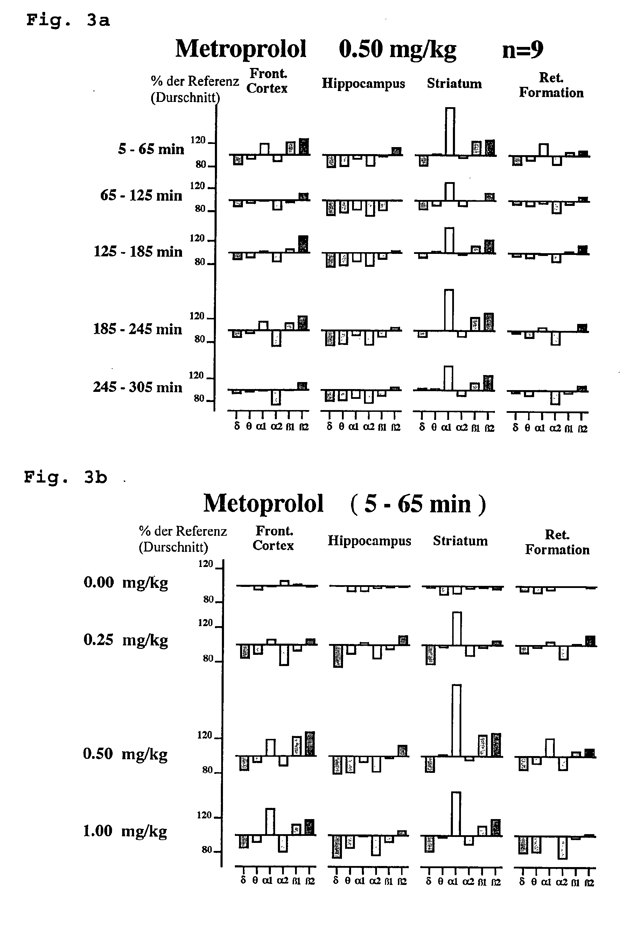

Metropolol zeigt überraschenderweise im Modell Tele-Stereo EEG bei Ratten Veränderungen der EEG-Frequenzen, wie sie nach Gabe klassischer Neuroleptika, insbesondere nach Gabe von Haloperidol in diesem Modell beschrieben wurden (W. Dimpfel, M. Spüler, K. Wessel "Different neuroleptics show common dose and time dependent effects in quantitative field potential analysis in freely moving rats", Psychopharmacology (1992), 107, 195-202). Die Veränderungen der EEG-Frequenzen, wie sie nach Gabe klassischer Neuroleptika auftraten, sind in Fig. 1 gezeigt. Aus der in Fig. 2 gezeigten Diskriminanzanalyse ist offensichtlich, dass sich der charakteristische "Fingerprint", der für die klassischen Antidepressiva kennzeichnend ist, auch im Muster von Metropolol in signifikanter Weise wiederfindet. Im Vergleich dazu zeigt Fig. 3a und 3b die Veränderungen der EEG-Frequenzen nach Gabe von Metropolol wie sie gemäß Beispiel 1 gemessen wurden. Aus der Erkenntnis, dass Metropolol die gleichen charakteristischen Veränderungen der EEG-Frequenzen hervorruft wie übliche antipsychotisch wirksame Arzneimittel (Neuroleptika), kann gefolgert werden, dass Metropolol bei der Behandlung von Schizophrenie wirksam und nützlich ist.

Erfindungsgemäß wird Metropolol eingesetzt. Diese Substanz besitzt die folgende Formel:

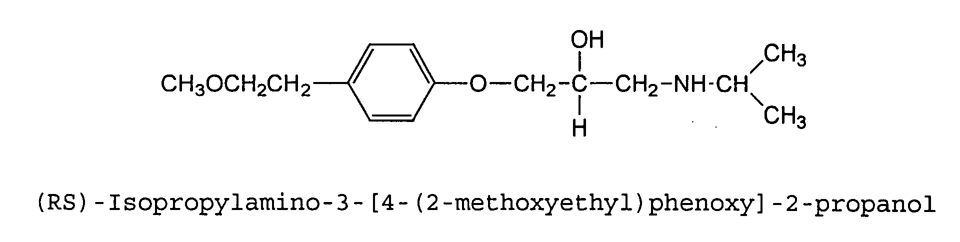

Metropolol kann erfindungsgemäß auch in Form seiner pharmazeutisch annehmbaren Salze eingesetzt werden. Die Herstellung solcher Salze erfolgt in an sich bekannter Weise. Als Salzbildner kommen alle üblichen pharmazeutisch annehmbaren Säuren bzw. Anionen in Frage. Beispiele für solche Salze schließen übliche Säureadditionssalze mit z.B. Salzsäure, Bromwasserstoffsäure, Schwefelsäure, Zitronensäure, Weinsäure, Phosphorsäure, Milchsäure, Brenztraubensäure, Essigsäure, Bernsteinsäure, Fumarsäure, Maleinsäure, Oxalessigsäure, Methansulfonsäure, Ethansulfonsäure, p-Toluolsulfonsäure, Benzolsulfonsäure und Isethionsäure ein. Bevorzugte Salze des Metropolols sind das Succinat, das Tartrat und das Hemitartrat. Besonders bevorzugt ist das Tartrat.

Die erfindungsgemäßen Metropolol als Wirkstoff enthaltenden Arzneimittel werden bevorzugt oral verabreicht. Es ist jedoch auch eine Verabreichung auf anderem Wege, z.B. peroral, topisch, parenteral, intravenös, intramuskulär, subkutan, nasal, inhalativ, rektal, transdermal möglich.

Zur Verabreichung kann das erfindungsgemäße Arzneimittel, das als Wirkstoff Metropolol enthält, z.B. in Form von Tabletten, Kapseln, Pillen, Dragees, Granulaten, Suppositorien, Pellets, Lösungen oder Dispersionen formuliert werden, wobei der Wirkstoff optional mit pharmazeutisch annehmbaren Hilfs- und Trägerstoffen kombiniert werden kann.

Liegt das erfindungsgemäße Arzneimittel in Form einer Lösung vor, so enthält diese bevorzugt 0,5 bis 20 Gew.-%, besonders bevorzugt 1 bis 10 Gew.-% des Wirkstoffs.

Die erfindungsgemäßen Arzneimittel, die Metropolol als Wirkstoff enthalten, werden normalerweise gemäss herkömmlichen Methoden hergestellt und in einer pharmazeutisch geeigneten Form verabreicht.

Zum Beispiel können die festen oralen Formen zusammen mit dem Wirkstoff Streckstoffe, z.B. Lactose, Dextrose, Saccharose, Cellulose, Maisstärke oder Kartoffelstärke; Gleitmittel, z.B. Silicat, Talk, Stearinsäure, Magnesium- oder Calciumstearat und/oder Polyethylenglykole; Bindemittel, z.B. Stärken, Gummi arabicum, Gelatine, Methylcellulose, Carboxymethylcellulose oder Polyvinylpyrrolidon; Aufschlussmittel, z.B. Stärke, Alginsäure, Alginate oder Natriumstärkeglykolate, aufschäumende Mischungen; Farbstoffe; Süßungsmittel; Benetzungsmittel, wie Lecithin, Polysorbate, Laurylsulfate; und im allgemeinen nicht-toxische und pharmakologisch inaktive Substanzen, die in pharmazeutischen Formulierungen verwendet werden, enthalten.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen zur oralen Verabreichung können z.B. Sirupe, Emulsionen und Suspensionen sein.

Der Sirup kann als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pectin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbaren Träger, z.B. steriles Wasser, Olivenöl, Ethyloleat, Glykole, z.B. Propylenglykol, und, falls gewünscht, eine geeignete Menge an Lidocain-Hydrochlorid enthalten.

Die Lösungen zur intravenösen Injektion oder Infusion können als Träger z.B. steriles Wasser enthalten oder sie können bevorzugt in Form von sterilen, wässrigen, isotonischen Salzlösungen vorliegen.

Die Suppositorien können zusammen mit dem Wirkstoff einen pharmazeutisch annehmbaren Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Die Dosierungseinheit des Arzneimittels kann beispielsweise enthalten:

bei peroralen Arzneiformen:
bevorzugt 20 bis 250 mg, besonders bevorzugt 50 bis 100 mg, Wirkstoff pro Einzeldosis. Die Tagesdosis kann beispielsweise in 1 bis 3 Einzeldosen, vorzugsweise in einer Einzeldosis, täglich verabreicht werden. bei parenteralen Arzneiformen (zum Beispiel intravenös, intramuskulär):
bevorzugt 1 bis 20 mg, besonders bevorzugt 5 bis 10 mg, Wirkstoff pro Einzeldosis. Die Tagesdosis kann beispielsweise in 1 bis 3 Einzeldosen, vorzugsweise in einer Einzeldosis, täglich verabreicht werden.

bei Arzneiformen zur rektalen Applikation:
100 bis 300 mg, vorzugsweise 200 mg Wirkstoff, pro Einzeldosis. Die Tagesdosis kann beispielsweise in 1 bis 3 Einzeldosen, vorzugsweise in 2 Einzeldosen, täglich verabreicht werden.

bei Arzneiformen zur Applikation auf die Haut und Schleimhäute (z.B. Lösungen, Lotionen, Emulsionen, Salben usw.):
100 bis 300 mg Wirkstoff, vorzugsweise 200 mg, pro Einzeldosis. Die Tagesdosis kann beispielsweise in 1 bis 6, vorzugsweise 1 bis 4, insbesondere 1 bis 3, Einzeldosen täglich verabreicht werden.

Bei Verwendung eines pharmazeutische annehmbaren Salzes muss die verwendete Menge in dem Fachmann bekannter Weise entsprechend angepasst werden.

### Testverfahren (Tele-Stereo-EEG)

Die Veränderungen der EEG-Frequenzen nach Gabe von Salzlösung (Kontrolle) bzw. einer Dosis von Metropolol (0,25 bis 1 mg/kg Körpergewicht) wurden bestimmt.

Die Untersuchungen wurden analog der durch W. Dimpfel et al. beschriebenen Methode (W. Dimpfel, M. Spüler, K. Wessel "Different neuroleptics show common dose and time dependent effects in quantitative field potential analysis in freely moving rats", Psychopharmacology (1992), 107, 195-202) folgendermaßen durchgeführt:

8 Männlichen erwachsenen Fischer-344 Ratten (Tag-Nacht konvertiert) wurden im Alter von 4 bis 5 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Computer System (Software "EEG-Analyse", Betriebsystem OS Science, Laborrechner "LabTeam" der Firma MediSyst, Linden DE) in Echtzeit einer Fast-Fourier-Transformation unterworfen und die Leistungsdichtespektren jeweils über 15 bis 60 Minuten gemittelt. Die Unterteilung der Spektren in 6 verschiedene Frequenzbereiche erlaubte die Erfassung pharmakospezifischer Veränderungen in Bezug auf die jeweils vor der Applikation gemessenen Vorwerte innerhalb dieser Frequenzbänder.

Zum Applikationsprotokoll der Substanzen: Die Substanzen wurden i.p. 45 Minuten nach Beginn der Messungen (Vorwert) injiziert. Fünf Minuten später wurden die Messungen wieder gestartet, mindestens über die nächsten 5 Stunden kontinuierlich analysiert und in 15-minütigen Perioden zusammengefasst. Die Testsubstanz wurde in einer Dosierung von 0,25 bis 1 mg/kg appliziert. Die experimentelle Serie wurde mit der Injektion von Salzlösung (Kontrolle) begonnen.

Der statistische Vergleich der Versuche erfolgte mit Hilfe einer multivariaten Analyse nach Ahrens und Läuter (siehe H. Ahrens, J. Läuter "Mehrdimensionale Varianzanalyse" (1974), Akademie Verlag, Berlin) auf der Basis der Veränderungen innerhalb der einzelnen Frequenzbänder in allen Hirnregionen als Variablen.

Die Verabreichung von Salzlösung führte nur zu geringfügigen Veränderungen in der elektrischen Aktivität (µV2/Ω) im Vergleich zu den Vorphasenwerten.

Bereits in der ersten Stunde nach Verabreichung von Metropolol wurde (außer im Hippokampus) eine deutliche Zunahme der alphal- sowie beta-Frequenzen beobachtet. Während der nächsten Stunde dominierte der Abfall einiger Frequenzen das Spektrum der Veränderungen auf eine sehr charakteristische Weise, wobei die Zunahme im alpha1-Bereich sich vorwiegend nur noch auf das Striatum erstreckte (Fig. 3a). Die Veränderungen waren statistisch mindestens signifikant auf dem P < 5 %-Level.

Die i.p. Verabreichung von Metropolol als Einzeldosis führt zu Veränderungen der elektrischen Gehirnaktivität bei den Testtieren, die ein statistisch signifikantes Niveau im Vergleich zu Salzlösung (0,9 % NaCl-Lösung) in allen Hirngebieten und bei allen Frequenzen erreichen. Diese Muster korrelieren in signifikanter Weise mit den Mustern, die für andere neuroleptisch wirksame Arzneimittel kennzeichnend sind ("Fingerprint") (Fig. 1); nicht jedoch mit den Mustern, die bei Medikamenten für andere Indikationen auftreten.

Die beobachteten Frequenzänderungen wurden mittels einer Diskriminanzanalyse mit den Ergebnissen mit herkömmlichen antipsychotisch wirkenden Arzneimitteln sowie Arzneimitteln für andere Indikationen verglichen. Es wurden folgende Arzneimittel verwendet: 0,5 mg/kg Metropolol (Metropr), 2 mg/kg Paroxetin (Parox), 10 mg/kg Imipramin (Imip), 10 mg/kg Amitryptilin (Amit), 1 mg/kg Nomifensin (Nomi), 0.5 mg/kg Midazolam (Midaz), 60 mg/kg Phenobarbital (Pheno), 60 mg/kg Meprobamat (Meprob), 0.5 mg/kg Chlorpromazin (Chlorp), 0,25 mg/kg Haloperidol (Halop), 1 mg/kg Prothipendyl (Prothi), 10 mg/kg Thioridazin (Thior), 15 mg/kg Carbamazepin (Carba), 75 mg/kg Valproinsäure (Valpr), 6 mg/kg Phenytoin (Pheny). Des weiteren wurden geprüft: 1,3 Vol.-% Halothan (Haloth), 100 mg/kg Propofol (Propo), 1,0 Vol.-% Isofluran (Isofl), 4,5 Vol.-% Desfluran (Desfl), 1,8 Vol.-% Sevofluran (Sevofl) und 1,7 Vol.-% Enfluran (Enfl). Die Ergebnisse sind in Fig. 2 dargestellt.

## Patentansprüche

1. Verwendung von Metropolol oder seinen pharmazeutisch annehmbaren Salzen zur Herstellung eines Arzneimittels zur Behandlung von Krankheiten des schizophrenen Formenkreises.

2. Verwendung nach Anspruch 1, **dadurch gekennzeichnet, dass** das Arzneimittel zusätzlich pharmazeutisch annehmbare Träger, Hilfs- und/oder Verdünnungsmittel enthält.

3. Verwendung nach einem der Ansprüche 1 oder 2, **dadurch gekennzeichnet, dass** das Arzneimittel zur oralen Verabreichung hergerichtet wird.
